# FASCICULE DE BREVET EUROPEEN

(11) **EP 2 044 976 B1**
(45) Date de publication et mention de la délivrance du brevet: **27.02.2013**
(21) Numéro de dépôt: 08158657.0
(22) Date de dépôt: 20.06.2008
(51) Int. Cl.: A61Q 1/06, A61K 8/88, A61K 8/898, A61K 8/97, A61Q 1/00, A61Q 19/00

(54) **Composition cosmétique associant un polymère siliconé et une résine tackifiante, et présentant une certaine élasticité**
Kosmetische Zusammensetzung, die ein Silikonpolymer und ein klebrigmachendes Harz enthält und eine gewisse Elastizität aufweist
Cosmetic composition combining a silicone polymer and a tackifying resin, and having a certain degree of elasticity

(30) Priorité: 03.07.2007 FR 0756248
(43) Date de publication de la demande: 08.04.2009
(73) Titulaire: L'Oréal, 75008 Paris (FR)
(72) Inventeur: Ilekti, Philippe, 94700 Maison-Alfort (FR); Le Chaux, Laure, 94240 L'Hay les Roses (FR)
(74) Mandataire: Boulard, Denis

(56) Documents cités:
- WO-A-2007/008575
- WO-A-2007/031872
- US-A1- 2004 180 020

## Description

La présente invention se rapporte à une composition cosmétique solide, notamment de maquillage et/ou de soin destinée à être appliquée sur la peau, les lèvres ou les phanères des êtres humains comme les cheveux, les cils, les sourcils ou les ongles, comprenant au moins une phase grasse liquide comprenant au moins un polyamide siliconé et au moins une résine tackifiante, telle que décrite dans la revendication 1.

Dans les produits cosmétiques ou dermatologiques, il est courant de trouver une phase liquide structurée, à savoir gélifiée et/ou rigidifiée. Ceci est notamment le cas dans les compositions solides, en particulier les compositions coulées solides, les baumes et les rouges à lèvres, les fards à paupières, les produits anti-cernes et les fonds de teint coulés. Cette structuration est classiquement obtenue à l'aide de cires ou de charges ou plus récemment à partir de gélifiants spécifiques.

Ainsi, dans les documents WO-A-97/36573, US-A-5 874 069, US-A-5 919 441, US-A-6 051 216, WO-A-02/17870, WO-A-02/17871, EP-A-1 177 784, WO-A-99/06473, et US-A-6 353 076 qui est une division du US-A-6 051 216, ainsi que dans le document EP 1 695 693 sont proposées des compositions cosmétiques telles que des sticks ou des gels de déodorant, comprenant une phase huileuse siliconée gélifiée par un polymère siliconé de type polysiloxane/polyamide.

L'utilisation de ces polymères siliconés permet d'accéder à une texture solide originale de composition cosmétique, à savoir dotée d'une rigidité relativement faible et d'une élasticité élevée. Cette texture ne correspond ni à celle d'un stick conventionnel doté d'une rigidité assez élevée, ni à celle d'un gel classique dont la consistance est liquide ou pâteuse.

Conjointement à cet ajustement de texture, les inventeurs ont cherché à accéder à une formulation de composition qui soit en outre dotée de bonnes qualités de déformabilité pour une application aisée et agréable et qui puisse donner également, si besoin est, satisfaction en terme de brillance.

En outre, ils ont cherché à obtenir une composition présentant une bonne stabilité, et en particulier de limiter les phénomènes d'exsudation, notamment dans des atmosphères chaudes.

De plus, on observe de manière inattendue que l'association d'au moins un polyamide siliconé et d'une résine tackifiante particulière permet l'obtention d'une composition présentant une résistance améliorée lors de l'application, en particulier d'éviter que celle-ci ne se fracture lorsqu'on utilise.

De manière inattendue, les inventeurs ont constaté que l'utilisation d'une résine tackifiante particulière en association avec un tel polyamide siliconé permettait d'obtenir des compositions donnant satisfaction en ces termes.

En conséquence, la présente invention concerne selon un premier aspect, une composition cosmétique solide de maquillage ou de soin comprenant dans un milieu physiologiquement acceptable au moins une phase grasse liquide comprenant :
- au moins une résine hydrocarbonée indénique de poids moléculaire moyen en nombre inférieur ou égal à 10000 g/mol,
- au moins un polyamide siliconé,
ledit polyamide siliconé comprenant une portion siliconée présentant un degré de polymérisation moyen supérieur ou égal à 50 et représentant au moins 10% du poids total de ladite composition, au moins une huile, ladite composition étant exempte d'huile volatile.

Le terme « solide » caractérise l'état de la composition à température ambiante (25°C) et à pression atmosphérique (760 mm de Hg).

De façon encore préférée, le polyamide siliconé représente au moins 15%, ou mieux au moins 20%, du poids total de ladite composition.

De façon préférée, la composition présente une élasticité supérieure à 80%. En effet, l'association de ladite résine et d'au moins un polyamide siliconé dont le degré de polymérisation moyen d'une portion siliconée est supérieur ou égal à 50 et représentant au moins 10%, ou mieux 15%, ou encore mieux 20% en poids de ladite composition permet l'obtention d'une composition présentant une élasticité supérieure à 80%, en particulier supérieure 90%.

L'invention permet d'obtenir des compositions ayant un glissant amélioré et leur application est agréable. Le dépôt obtenu est homogène et présente une brillance améliorée.

La présente invention a également pour objet un procédé de maquillage ou de soin des matières kératiniques et notamment des lèvres dans lequel on applique sur les lèvres une composition telle que définie précédemment.

Elle concerne de même selon un autre de ses aspects, l'utilisation d'un polyamide siliconé tel que défini précédemment en association avec ladite résine pour préparer une composition cosmétique, stable et apte à procurer un film de brillance améliorée.

De manière inattendue, les compositions solides selon l'invention s'avèrent ainsi dotées d'une texture élastique qui peut être associée à une gestuelle d'application particulière.

Par exemple, les textures solides souples et élastiques obtenues selon l'invention peuvent être compatibles avec une application directe, sur les lèvres par exemple, sans requérir l'utilisation d'applicateur comme dans le cas de textures fluides. Les compositions selon l'invention manifestent en outre lors de leur application, une souplesse, une douceur et une très bonne élasticité préservant ainsi le produit pour une prochaine application. Le produit peut ne pas se déformer de façon définitive et reprendre sa forme initiale après application.

Sous la forme d'un dôme, les compositions conformes à l'invention manifestent avantageusement le comportement d'un solide élastique déformable et souple, conférant à l'application une douceur remarquable.

De plus, les compositions selon l'invention présentent un glissant et un délitage amélioré lors de l'application, qui est dès lors plus agréable.

### Caractérisation de l'élasticité et de la dureté

Avantageusement, les compositions selon l'invention possèdent une dureté variant de 10 à 250 g et/ou une élasticité supérieure à 80 %.

La dureté et l'élasticité de la composition selon l'invention peuvent être mesurées à l'aide d'un texturomètre qui permet d'obtenir la variation de la résistance à la déformation de la composition en fonction du déplacement d'un mobile dans un échantillon de ladite composition.

Le texturomètre mesure la force de résistance à la déformation de la composition dès que le mobile entre en contact avec l'échantillon. Après avoir atteint une profondeur maximale programmée L0 dans l'échantillon, le mobile retourne au point initial.

La dureté (exprimée en gramme ou en Newton) est égale à la valeur de résistance de la composition lorsque le mobile est en bout de course, et l'élasticité (exprimée en pourcentage) est égale au rapport de i) la distance L à laquelle se produit la rupture de contact entre le mobile et l'échantillon au cours du retrait du mobile et ii) de la distance L0. La rupture de contact se traduit par l'annulation de la force de résistance de la composition sur le mobile.

L'élasticité est proportionnelle à la distance sur laquelle le système va « accompagner » la remontée du mobile ; plus elle est importante, plus le système est élastique.

Le texturomètre utilisé peut notamment être un texturomètre Stable Micro System TAX-T2i^{®} équipé du logiciel d'exploitation type Texture Expert Exceed^{®} muni d'un mobile plastique hémisphérique P/0.5 HS Rheo's.

Les paramètres appliqués sont avantageusement les suivants :
- vitesse avant contact : 0,1 mm.s-1,
- vitesse de déplacement dans l'échantillon : 0,1 mm.s-1,
- vitesse de retrait : 0,1 mm.s-1,
- profondeur maximale L0 : 1 mm.

On prépare les échantillons de composition en coulant à chaud une quantité suffisante de la composition à tester, par exemple dans une boite de Pétri 100 x 15 mm préalablement tarée, pour obtenir un échantillon d'environ 1 cm d'épaisseur. L'intérêt du choix de ce conditionnement est sa largeur suffisante pour s'affranchir de tout effet de bord. On prépare ainsi deux boîtes de Pétri, qui sont laissées au repos un minimum de 24 h à 20 °C avant caractérisation.

Au minimum trois mesures sont réalisées sur chaque échantillon (boîte de pétri): une, au centre et deux autres, sur des points situés à équidistance du centre et du bord de celle-ci.

La dureté et l'élasticité sont égales à la moyenne des mesures effectuées.

La dureté de la composition selon l'invention est telle que la composition est autoportée et peut se déliter aisément pour former un dépôt satisfaisant sur la peau et les lèvres. En outre, avec cette dureté, la composition de l'invention résiste bien aux chocs.

Plus particulièrement, la dureté peut varier de 30 g à 200 g, notamment de 50 g à 190 g, voire de 70 à 175 g et plus particulièrement de 100 g à 150 g.

Les compositions selon l'invention possèdent avantageusement une élasticité supérieure à 90 %.

### Caractérisation de la stabilité

On prépare des échantillons de la composition en coulant à chaud (environ 100°C) une quantité suffisante de la composition à tester, par exemple dans une boite de Pétri 100x15 mm, pour obtenir un échantillon d'environ 1 cm d'épaisseur. La boite de Pétri est laissé au repos un minimum de 24h à 20°C. Les échantillons sont ensuite conservés à température ambiante d'une part et à 47°C d'autre part pendant deux mois.

L'exsudation se traduit par l'apparition de gouttelette d'huile à la surface de l'échantillon.

Ce test permet d'évaluer la stabilité de la composition à température ambiante et à 47°C.

### Polyamide siliconé

Comme indiqué précédemment, les compositions selon l'invention comprennent au moins un polyamide siliconé.

Les polyamides siliconés de la composition sont de préférence solides à la température ambiante (25 °C) et pression atmosphérique (760 mm de Hg).

Par polymère, on entend au sens de l'invention un composé ayant au moins 2 motifs de répétition, de préférence au moins 3 motifs de répétition et mieux encore 10 motifs de répétition.

Les polyamides siliconés de la composition de l'invention peuvent être des polymères du type polyorganosiloxane comme par exemple ceux décrits dans les documents US-A-5 874 069, US-A-5,919,441, US-A-6,051,216 et US-A-5,981,680. Selon l'invention, les polymères siliconés peuvent appartenir aux deux familles suivantes :
(1) des polyorganosiloxanes comportant au moins deux groupes amides, ces deux groupes étant situés dans la chaîne du polymère, et/ou
(2) des polyorganosiloxanes comportant au moins deux groupes amides, ces deux groupes étant situés sur des greffons ou ramifications.

A) Selon une première variante, les polymères siliconés sont des polyorganosiloxanes tels que définis ci-dessus et dont les motifs amides sont disposés dans la chaîne du polymère.

Les polyamides siliconés peuvent plus particulièrement être des polymères comprenant au moins un motif répondant à la formule générale I : 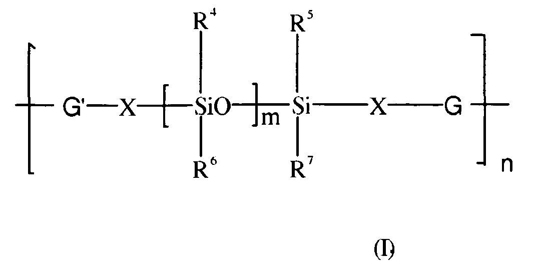
1) dans laquelle : G' représente C(O) quand G représente -C(O)-NH-Y-NH-, et G' représente -NH- quand G représente -NH-C(O)-Y-C(O)-
2) R⁴, R⁵, R⁶ et R⁷, identiques ou différents, représentent un groupe choisi parmi :
   - les groupes hydrocarbonés, linéaires, ramifiés ou cycliques, en C₁ à C₄₀, saturés ou insaturés, pouvant contenir dans leur chaîne un ou plusieurs atomes d'oxygène, de soufre et/ou d'azote, et pouvant être substitués en partie ou totalement par des atomes de fluor,
   - les groupes aryles en C₆ à C₁₀, éventuellement substitués par un ou plusieurs groupes alkyle en C₁ à C₄,
   - les chaînes polyorganosiloxanes contenant ou non un ou plusieurs atomes d'oxygène, de soufre et/ou d'azote,
3) les X, identiques ou différents, représentent un groupe alkylène di-yle, linéaire ou ramifié en C₁ à C₃₀, pouvant contenir dans sa chaîne un ou plusieurs atomes d'oxygène et/ou d'azote,
4) Y est un groupe divalent alkylène linéaire ou ramifié, arylène, cycloalkylène, alkylarylène ou arylalkylène, saturé ou insaturé, en C₁ à C₅₀, pouvant comporter un ou plusieurs atomes d'oxygène, de soufre et/ou d'azote, et/ou porter comme substituant l'un des atomes ou groupes d'atomes suivants : fluor, hydroxy, cycloalkyle en C₃ à C₈, alkyle en C₁ à C₄₀, aryle en C₅ à C₁₀, phényle éventuellement substitué par 1 à 3 groupes alkyle en C₁ à C₃, hydroxyalkyle en C₁ à C₃ et amino alkyle en C₁ à C₆, ou
5) Y représente un groupe répondant à la formule : 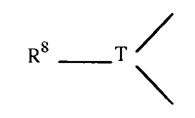 dans laquelle
   - T représente un groupe hydrocarboné trivalent ou tétravalent, linéaire ou ramifié, saturé ou insaturé, en C₃ à C₂₄ éventuellement substitué par une chaîne polyorganosiloxane, et pouvant contenir un ou plusieurs atomes choisis parmi O, N et S, ou T représente un atome trivalent choisi parmi N, P et Al, et
   - R⁸ représente un groupe alkyle en C₁ à C₅₀, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,
6) n est un nombre entier allant de 2 à 500, de préférence de 2 à 200, et m est un nombre entier allant de 50 à 1000, de préférence de 50 à 700 et mieux encore de 50 à 200.

On notera que « m » correspond au degré de polymérisation moyen de la portion siliconée du polyamide siliconé.

Selon un mode de réalisation de l'invention, 80 % des R⁴, R⁵, R⁶ et R⁷, du polymère sont choisis de préférence parmi les groupes méthyle, éthyle, phényle et 3,3,3-trifluoropropyle. Selon un autre mode, 80 % des R⁴, R⁵, R⁶ et R⁷, du polymère sont des groupes méthyle.

Selon l'invention, Y peut représenter divers groupes divalents, comportant éventuellement de plus une ou deux valences libres pour établir des liaisons avec d'autres motifs du polymère ou copolymère. De préférence, Y représente un groupe choisi parmi :
a) les groupes alkylène linéaires en C₁ à C₂₀, de préférence en C₁ à C₁₀,
b) les groupes alkylène ramifiés pouvant comporter des cycles et des insaturations non conjuguées, en C₃₀ à C₅₆,
c) les groupes cycloalkylène en C₅-C₆,
d) les groupes phénylène éventuellement substitués par un ou plusieurs groupes alkyle en C₁ à C₄₀,
e) les groupes alkylène en C₁ à C₂₀, comportant de 1 à 5 groupes amides,
f) les groupes alkylène en C₁ à C₂₀, comportant un ou plusieurs substituants, choisis parmi les groupes hydroxyle, cycloalcane en C₃ à C₈, hydroxyalkyle en C₁ à C₃ et alkylamines en C₁ à C₆,
g) les chaînes polyorganosiloxane de formule : 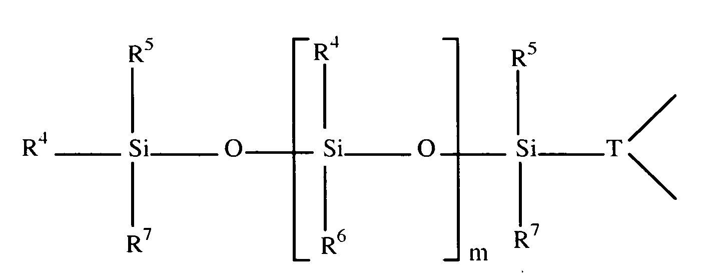 ou 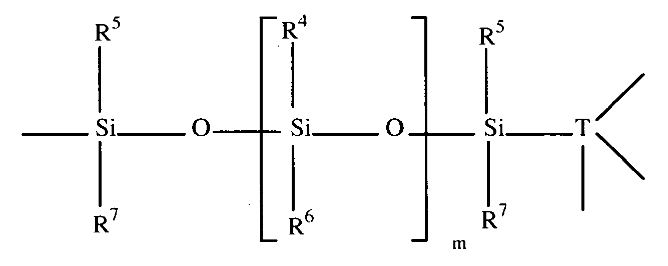 dans laquelle R⁴, R⁵, R⁶, R⁷, T et m sont tels que définis ci-dessus.

B) Selon la seconde variante, les polyamides siliconés peuvent être des polymères comprenant au moins un motif répondant à la formule (II) : 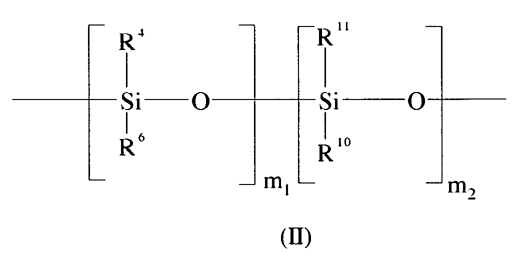 dans laquelle
- R⁴ et R⁶, identiques ou différents, sont tels que définis ci-dessus pour la formule (I),
- R¹⁰ représente un groupe tel que défini ci-dessus pour R⁴ et R⁶, ou représente le groupe de formule -X-G"-R¹² dans laquelle X sont tels que définis ci-dessus pour la formule (I) et R¹² représente un atome d'hydrogène ou un groupe hydrocarboné, linéaire, ramifié ou cyclique, saturé ou insaturé, en C₁ à C₅₀ comportant éventuellement dans sa chaîne un ou plusieurs atomes choisis parmi O, S et N, éventuellement substitué par un ou plusieurs atomes de fluor et/ou un ou plusieurs groupes hydroxyle, ou un groupe phényle éventuellement substitué par un ou plusieurs groupes alkyle en C₁ à C₄,
   et G" représente -C(O)NH- et -HN-C(O)-.
   R¹¹ représente le groupe de formule -X-G"-R¹² dans laquelle X, G" et R¹² sont tels que définis ci-dessus,
- m₁ est un nombre entier allant de 50 à 998, et
- m₂ est un nombre entier allant de 2 à 500.
On notera que « m₁ » correspond au degré de polymérisation moyen de la portion siliconée du polyamide siliconé.

Selon l'invention, le polymère siliconé peut être un homopolymère, c'est-à-dire un polymère comportant plusieurs motifs identiques, en particulier des motifs de formule (I) ou de formule (II).

Selon l'invention, on peut aussi utiliser un polymère constitué par un copolymère comportant plusieurs motifs de formule (I) différents, c'est-à-dire un polymère dans lequel l'un au moins des R⁴, R⁵, R⁶, R⁷, X, G, Y, m et n est différent dans l'un des motifs. Le copolymère peut être aussi formé de plusieurs motifs de formule (II), dans lequel l'un au moins des R⁴, R⁶, R¹⁰, R¹¹, m₁ et m₂ est différent dans l'un au moins des motifs.

On peut encore utiliser un polymère comportant au moins un motif de formule (I) et au moins un motif de formule (II), les motifs de formule (I) et les motifs de formule (II) pouvant être identiques ou différents les uns des autres.

Selon une variante de l'invention, on peut encore utiliser un polyamide siliconé comprenant de plus au moins un motif hydrocarboné comportant deux groupes capables d'établir des interactions hydrogènes choisis parmi les groupes ester, amide, sulfonamide, carbamate, thiocarbamate, urée, uréthane, thiourée, oxamido, guanidino, biguanidino et leurs combinaisons.

Ces copolymères peuvent être des polymères blocs, des polymères séquencés ou des polymères greffés.

Dans les formules (I) et (II), le groupe alkylène représentant X ou Y peut éventuellement contenir dans sa partie alkylène au moins l'un des éléments suivants :
1) 1 à 5 groupes amides, urée, uréthane, ou carbamate,
2) un groupe cycloalkyle en C₅ ou C₆, et
3) un groupe phénylène éventuellement substitué par 1 à 3 groupes alkyles identiques ou différents en C₁ à C₃.

Dans les formules (I) et (II), les groupes alkylènes peuvent aussi être substitués par au moins un élément choisi dans le groupe constitué de :
- un groupe hydroxy,
- un groupe cycloalkyle en C₃ à C₈,
- un à trois groupes alkyles en C₁ à C₄₀,
- un groupe phényle éventuellement substitué par un à trois groupes alkyles en C₁ à C₃,
- un groupe hydroxyalkyle en C₁ à C₃, et
- un groupe aminoalkyle en C₁ à C₆.

Dans ces formules (I) et (II), Y peut aussi représenter : 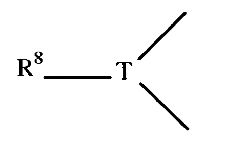 où R⁸ représente une chaîne polyorganosiloxane, et T représente un groupe de formule : 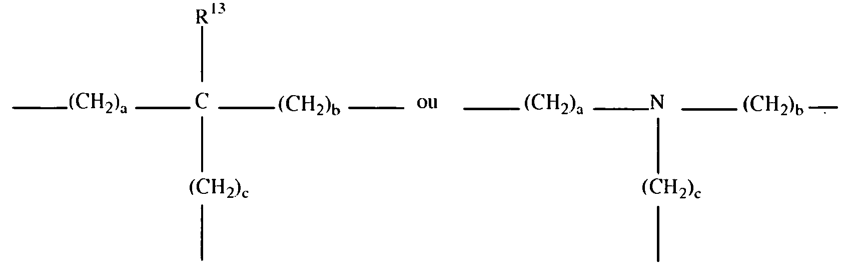 dans lesquelles a, b et c sont indépendamment des nombres entiers allant de 1 à 10, et R¹³ est un atome d'hydrogène ou un groupe tel que ceux définis pour R⁴, R⁵, R⁶ et R⁷.

Dans les formules (I) et (II), R⁴, R⁵, R⁶ et R⁷ représentent de préférence, indépendamment, un groupe alkyle en C₁ à C₄₀, linéaire ou ramifié, de préférence un groupe CH₃, C₂H₅, n-C₃H₇ ou isopropyle, une chaîne polyorganosiloxane ou un groupe phényle éventuellement substitué par un à trois groupes méthyle ou éthyle.

Comme on l'a vu précédemment, le polymère peut comprendre des motifs de formule (I) ou (II) identiques ou différents.

Ainsi, le polymère peut être un polyamide contenant plusieurs motifs de formule (I) ou (II) de longueurs différentes, soit un polyamide répondant à la formule (III) : 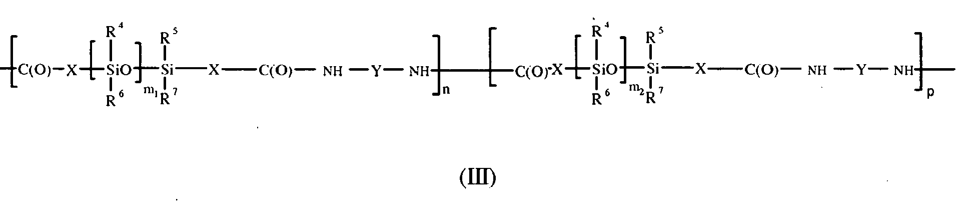 dans laquelle X, Y, n, R⁴ à R⁷ ont les significations données ci-dessus, m₁ et m₂ qui sont différents, sont choisis dans la gamme allant de 1 à 1000, et p est un nombre entier allant de 2 à 300.

Dans cette formule, les motifs peuvent être structurés pour former soit un copolymère bloc, soit un copolymère aléatoire, soit un copolymère alterné. Dans ce copolymère, les motifs peuvent être non seulement de longueurs différentes mais aussi de structures chimiques différentes, par exemple ayant des Y différents. Dans ce cas, le polymère peut répondre à la formule IV: 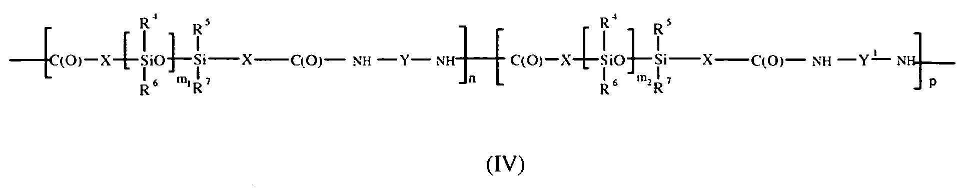 dans laquelle R⁴ à R⁷, X, Y, m₁, m₂, n et p ont les significations données ci-dessus et Y¹ est différent de Y mais choisi parmi les groupes définis pour Y. Comme précédemment, les différents motifs peuvent être structurés pour former soit un copolymère bloc, soit un copolymère aléatoire, soit un copolymère alterné.

Dans ce premier mode de réalisation de l'invention, le polymère siliconé peut être aussi constitué par un copolymère greffé. Ainsi, le polyamide à unités silicone peut être greffé et éventuellement réticulé par des chaînes silicones à groupes amides. De tels polymères peuvent être synthétisés avec des amines trifonctionnelles.

Selon l'invention, comme on l'a vu précédemment, les unités siloxanes peuvent être dans la chaîne principale ou squelette du polymère, mais elles peuvent également être présentes dans des chaînes greffées ou pendantes. Dans la chaîne principale, les unités siloxanes peuvent être sous forme de segments comme décrits ci-dessus. Dans les chaînes pendantes ou greffées, les unités siloxanes peuvent apparaître individuellement ou en segments.

Selon une variante de réalisation de l'invention, on peut utiliser un copolymère de polyamide silicone et de polyamide hydrocarboné, soit un copolymère comportant des motifs de formule (I) ou (II) et des motifs polyamide hydrocarboné. Dans ce cas, les motifs polyamide-silicone peuvent être disposés aux extrémités du polyamide hydrocarboné.

Avantageusement, la composition comprend au moins un polymère polyamide/polydiméthylsiloxane, notamment un polymère de formule générale (I) possédant un indice m de valeur supérieure à 50, en particulier supérieure à 75, notamment d'environ 100.

De façon avantageuse, le polyamide siliconé de formule (I) a une masse moléculaire moyenne en poids allant de 10 000 à 500 000 g/mol.

De préférence encore, X et Y représentent indépendamment un groupe choisi parmi les groupes alkylène linéaires en C₁ à C₂₀, de préférence en C₁ à C₁₀.

A titre d'exemples de polymère utilisable, on peut citer un des polyamides siliconés, obtenus conformément aux exemples 1 à 3 du document US-A-5 981 680, tel que le produit commercialisé sous la référence DC 2-8179 par Dow Corning).

Selon une variante de réalisation de l'invention, le polymère est constitué par un homopolymère ou copolymère comportant des groupes uréthane ou urée. Ces polymères sont décrits en détail dans la demande WO 2003/106614.

La première composition peut contenir à la place du polyamide siliconé un polymère polyorganosiloxane contenant deux ou plusieurs groupes uréthanes et/ou urées, soit dans le squelette du polymère, soit sur des chaînes latérales ou comme groupes pendants.

Les polymères comportant au moins deux groupes uréthanes et/ou urées dans le squelette peuvent être des polymères comprenant au moins un motif répondant à la formule suivante : 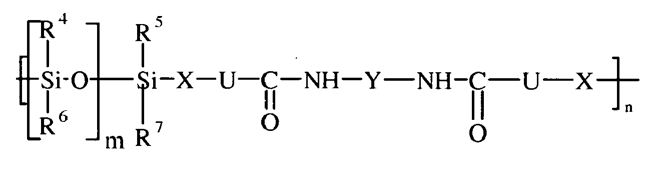 dans laquelle les R⁴, R⁵, R⁶, R⁷, X, Y, m et n ont les significations données ci-dessus pour la formule (I), et U représente -O- ou -NH-, afin que : 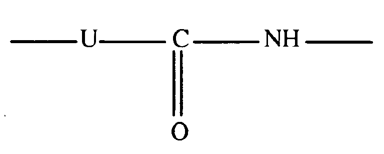 corresponde à un groupe uréthane ou urée.

Dans cette formule, Y peut être un groupe alkylène, en C₁ à C₄₀, linéaire ou ramifié, substitué éventuellement par un groupe alkyle en C₁ à C₁₅ ou un groupe aryle en C₅ à C₁₀. De préférence, on utilise un groupe -(CH₂)₆-.

Le polymère constituant le polymère siliconé peut être formé de motifs silicone uréthane et/ou silicone-urée de longueur et/ou de constitution différentes, et se présenter sous la forme de copolymères blocs, séquencés ou statistiques (aléatoires).

Comme dans le cas des polyamides silicones de formule (I), (II) ou (III), on peut utiliser dans l'invention des polyuréthanes ou des polyurées silicones ayant des motifs de longueur et de structure différentes, en particulier des motifs de longueurs différentes par le nombre d'unités silicones.

Les polymères et copolymères utilisés dans la composition de l'invention ont avantageusement une température de transition de l'état solide à l'état liquide allant de 45 °C à 190 °C. De préférence, ils présentent une température de transition de l'état solide à l'état liquide allant de 70 à 130 °C et mieux de 80 °C à 105 °C.

Le polyamide siliconé présent dans la première composition en une teneur totale allant de 10 % à 70 % en poids par rapport au poids total de la composition, de préférence allant de 10 % à 50 % en poids, et mieux allant de 15 % à 30 % en poids, de préférence allant de 20 à 30% en poids du poids total de ladite composition.

Selon un mode de réalisation avantageux, la composition comprend au moins deux polyamides siliconés, en particulier deux polyamides siliconés comprenant chacun indépendamment au moins un motif de formule (I). Les polyamides siliconés pourront par exemple présenter une portion siliconée présentant un degré de polymérisation moyen différent.

De façon préférée, selon ce mode de réalisation, la composition comprend :
- au moins un premier polyamide siliconé comprenant au moins un motif de formule (I) telle que définie précédemment dans laquelle m varie de 50 à 600, en particulier de 60 à 400, notamment de 75 à 200 et plus particulièrement est de l'ordre de 120 pour le premier polymère, et
- au moins un second polyamide siliconé comprenant au moins un second motif de formule (I) telle que définie précédemment dans laquelle G', G, R⁴, R⁵, R⁶, R⁷, X, Y et n sont tels que définis précédemment et m va de 5 à 100, en particulier de 10 à 75 et plus particulièrement est de l'ordre de 15 pour le second polymère.

De façon avantageuse, le premier polymère a une masse moléculaire moyenne en poids comprise entre de 10 000 et 500 000 g/mol. De façon avantageuse, le deuxième polymère a de préférence une masse moléculaire moyenne en poids allant de 1 000 à 500 000 g/mol, notamment entre 10 000 et 300 000 g/mol.

### Résine

La résine utilisée dans la composition selon l'invention a un poids moléculaire moyen en nombre inférieure ou égale à 10 000 g/mol, notamment allant de 250 à 10 000 g/mol de préférence inférieure ou égale à 5 000 g/mol, notamment allant de 250 à 5 000 g/mol, mieux, inférieure ou égale à 2 000 g/mol notamment allant de 250 à 2 000 g/mol et encore mieux inférieure ou égale à 1 000 g/mol notamment allant de 250 à 1 000 g/mol.

On détermine les poids moléculaires moyens en nombre (Mn) par chromatographie liquide par perméation de gel (solvant THF, courbe d'étalonnage établie avec des étalons de polystyrène linéaire, détecteur réfractométrique).

La résine de la composition selon l'invention est avantageusement une résine dite tackifiante. De telles résines sont notamment décrites dans le Handbook of Pressure Sensitive Adhesive, edited by Donatas Satas, 3rd ed., 1989, p. 609-619.

La résine de la composition selon l'invention est choisie parmi, les résines hydrocarbonées telles que décrites dans la revendication 1.

Les résines hydrocarbonées sont choisies parmi les
- résines hydrocarbonées indéniques telles que les résines issues de la polymérisation en proportion majoritaire de monomère indène et en proportion minoritaire de monomère choisi parmi le styrène, le méthylindène, le méthylstyrène et leurs mélanges. Ces résines pouvant éventuellement être hydrogénées. Ces résines peuvent présenter un poids moléculaire allant de 290 à 1150 g/mol.
   Comme exemples de résines indéniques, on peut citer celles commercialisées sous la référence ESCOREZ 7105 par la société Exxon Chem., NEVCHEM 100 et NEVEX 100 par la société Neville Chem., NORSOLENE S105 par la société Sartomer, PICCO 6100 par la société Hercules et RESINALL par la société Resinall Corp., ou les copolymères indène/méthylstyrène/styrène hydrogéné commercialisées sous la dénomination « REGALITE » par la société Eastman Chemical, en particulier REGALITE R 1100, REGALITE R 1090, REGALITE R-7100, REGALITE R1010 HYDROCARBON RESIN, REGALITE R1125 HYDROCARBON RESIN.

Selon un mode préféré de réalisation, la résine est choisie parmi résines hydrocarbonées indéniques en particulier les copolymères indène/méthylstyrène/styrène hydrogéné commercialisées sous la dénomination « REGALITE » par la société Eastman Chemical, tels que la REGALITE R 1100, REGALITE R 1090, REGALITE R-7100, REGALITE R1010 HYDROCARBON RESIN, REGALITE R1125 HYDROCARBON RESIN.

La résine tackifiante peut être présente dans la composition selon l'invention en une teneur allant de 0,1 à 30 % en poids, par rapport au poids total de la composition, de préférence allant de 0,3 à 20 % en poids, plus préférentiellement allant de 0,5 à 15 % en poids.

De façon préférée, la résine tackifiante est présente dans la composition dans un ratio tel que la proportion massique polyamide siliconé/ résine est supérieure à 2, et mieux, allant de 2 à 40.

### Phase grasse liquide

La phase grasse de la composition selon l'invention est en particulier une phase grasse liquide à base d'au moins une huile.

L'huile peut être une huile siliconée, une huile ester ou une huile non siliconée.

### a. Huile siliconée

Selon une variante de l'invention, la phase grasse liquide comprend au moins une huile siliconée. Celle-ci est une huile non volatile.

Au sens de l'invention, une huile non volatile présente à température ambiante (25 °C) et pression atmosphérique (760 mm de Hg) une pression de vapeur inférieure à 0,02 mm de Hg (2,66 Pa), et mieux, inférieure à 10⁻³ mm de Hg (0, 13 Pa).

Les huiles siliconées non volatiles peuvent être des polydiméthylsiloxanes, des polyalkylméthylsiloxanes, des diméthicone copolyols, des alkylméthicone copolyols, la cétyldiméthicone, des silicones à groupes alkylglycéryl éthers, des silicones à groupes amines latéraux et le dilauroyltriméthylol propane siloxysilicate. Les groupements alkyle de ces huiles ont notamment de 2 à 24 atomes de carbone.

Les huiles siliconées non volatiles utilisables dans la phase grasse liquide peuvent être en particulier les polydiméthylsiloxanes (PDMS) non volatils, linéaires, liquides à température ambiante ; les polydiméthylsiloxanes comportant des groupements alkyle, alcoxy ou phényle, pendants et/ou en bout de chaîne siliconée, groupements ayant chacun de 2 à 24 atomes de carbone ; les silicones fluorées à groupement(s) pendant(s) ou en bout de chaîne ayant de 1 à 12 atomes de carbone dont tout ou partie des atomes d'hydrogène est substitué par des atomes de fluor, les diméthiconols et leurs mélanges.

Selon un mode de réalisation préférée, la composition selon l'invention comprend en tant qu'huiles siliconées non volatiles une silicone phénylée. On entend par silicone phénylée, un organopolysiloxane substitué par au moins un groupe phényle.

La silicone phénylée est de préférence non volatile.

De préférence, le poids moléculaire de l'huile siliconée phénylée est compris entre 500 et 10 000 g/mol.

L'huile siliconée peut être choisie parmi les phényl triméthicones, les phényl diméthicones, les phényl triméthylsiloxy diphénylsiloxanes, les diphényl diméthicones, diphényl méthyldiphényl trisiloxanes.

L'huile siliconée peut répondre à la formule: 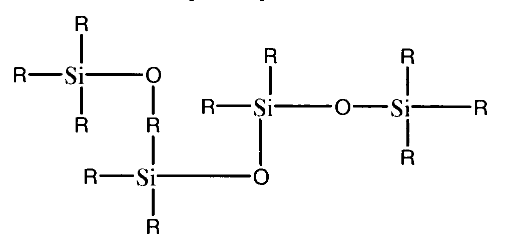 dans laquelle les groupements R représentent indépendamment les uns des autres un méthyle ou un phényle. De préférence dans cette formule, l'huile siliconée comprend au moins trois groupes phényle, par exemple au moins quatre, au moins cinq ou au moins six.

Selon un autre mode de mise en oeuvre, l'huile siliconée répond à la formule 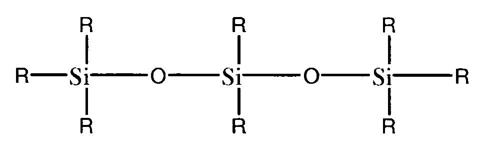 dans laquelle les groupements R représentent indépendamment les uns des autres un méthyle ou un phényle. De préférence dans cette formule, ledit organopolysiloxane comprend au moins trois groupes phenyls, par exemple au moins quatre ou au moins cinq. Des mélanges des organopolysiloxanes phénylés décrits précédemment peuvent être utilisés.

On peut citer par exemples des mélanges d' organopolysiloxane triphénylé, tétra- ou pentaphénylé.

Selon un autre mode de mise en oeuvre, l'huile siliconée répond à la formule 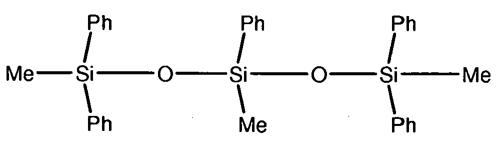 dans laquelle Me représente méthyle, Ph représente phényle. Une telle silicone phénylée est notamment fabriquée par Dow Corning sous la reference Dow Corning 555 Cosmetic Fluid (nom INCI : trimethyl pentaphenyl trisiloxane). La référence Dow Corning 554 Cosmetic Fluid peut aussi être utilisée.

Les huiles siliconées ont une viscosité choisie avantageusement dans la gamme allant de 5 à 800 000 cSt à 25 °C, de préférence de 10 à 500 000 cSt, et mieux de 10 à 5 000 cSt.

La phase grasse liquide contient avantageusement de 0,1 à 70 %, par exemple de 5 à 60 % en poids d'huile(s) siliconée(s).

De façon préférée, la ou les huiles siliconées représentent au moins 20% par rapport au poids total de la composition, ou mieux au moins 30%.

### b. Huile ester

Selon une variante de l'invention, au moins une des huiles de la phase grasse liquide est une huile, dite « huile ester », qui est choisie parmi les esters des acides monocarboxyliques avec les monoalcools et polyalcools.

Avantageusement, ledit ester répond à la formule suivante :

R₁-CO-O-R₂

où R₁ représente un radical alkyle linéaire ou ramifié de 1 à 40 atomes de carbone, de préférence de 7 à 19 atomes de carbone, comprenant éventuellement une ou plusieurs double liaisons éthyléniques, et éventuellement substitué.

R₂ représente un radical alkyle linéaire ou ramifié de 1 à 40 atomes de carbone, de préférence de 3 à 30 atomes de carbone et mieux de 3 à 20 atomes de carbone, comprenant éventuellement une ou plusieurs double liaisons éthyléniques, et éventuellement substitué.

Par « éventuellement substitué », on entend que R₁ et ou R₂ peuvent porter un ou plusieurs substituants choisis, par exemple, parmi les groupements comprenant un ou plusieurs hétéroatomes choisi parmi O, N et S, tels que amino, amine, alcoxy, hydroxyle.

De préférence, le nombre total d'atomes de carbone de R₁ + R₂ est ≥ 9.

R₁ peut représenter le reste d'un acide gras, linéaire ou de préférence ramifié comprenant de 1 à 40 et mieux de 7 à 19 atomes de carbone et R₂ peut représenter une chaîne hydrocarbonée linéaire ou de préférence ramifiée contenant de 1 à 40, de préférence de 3 à 30 et mieux de 3 à 20 atomes de carbone. De nouveau, de préférence, le nombre d'atomes de carbone de R₁ + R₂ ≥ 9.

Des exemples des groupes R₁ sont ceux dérivés des acides gras choisis dans le groupe constitué des acides acétique, propionique, butyrique, caproïque, caprylique, pélargonique, caprique, undécanoïque, laurique, myristique, palmitique, stéarique, isostéarique, arachidique, béhénique, oléique, linolénique, linoléïque, oléostéarique, arachidonique, érucique, et de leurs mélanges.

Des exemples d'esters utilisables dans les phases grasses des compositions de l'invention sont, par exemple, l'huile de purcellin (octanoate de cétostéaryle), l'isononanoate d'isononyle, le myristate d'isopropyle, le palmitate d'éthyl-2-hexyle, le stéarate d'octyl 2-dodécyle, l'érucate d'octyl 2-dodécyle, l'isostéarate d'isostéaryle, et les heptanoates, octanoates, décanoates ou ricinoléates d'alcools ou de polyalcools, par exemple d'alcools gras.

Avantageusement, les esters sont choisis parmi les composés de la formule ci-dessus, dans laquelle R₁ représente un groupe alkyle linéaire ou ramifié non substitué, comprenant éventuellement une ou plusieurs double liaisons éthyléniques, de 1 à 40 atomes de carbone, de préférence de 7 à 19 atomes de carbone, et R₂ représente un groupe alkyle linéaire ou ramifié non substitué, comprenant éventuellement une ou plusieurs double liaisons éthyléniques, de 1 à 40 atomes de carbone, de préférence de 3 à 30 atomes de carbone, et mieux de 3 à 20 atomes de carbone.

De préférence, R₁ est un groupe alkyle ramifié non substitué de 4 à 14 atomes de carbone, de préférence de 8 à 10 atomes de carbone et R₂ est un groupe alkyle ramifié non substitué de 5 à 15 atomes de carbone, de préférence de 9 à 11 atomes de carbone. De préférence dans la formule (I), R₁-CO- et R₂ ont le même nombre d'atomes de carbone et dérivent du même radical, de préférence alkyle ramifié non substitué, par exemple isononyle, c'est-à-dire que avantageusement la molécule d'huile ester est symétrique.

L'huile ester sera choisie, de préférence, parmi les composés suivants :
- l'isononanoate d'isononyle,
- l'octanoate de cétostéaryle,
- le myristate d'isopropyle,
- le palmitate d'éthyl-2-hexyle,
- le stéarate d' octyl 2-dodécyle,
- l'érucate d'octyl 2-dodécyle,
- l'isostéarate d'isostéaryle.

L'ester préféré entre tous est l'isononanoate d'isononyle.

Selon un mode de réalisation, la composition est exempte d'huile ester volatile.

Avantageusement, la phase grasse liquide comprend de 0,1 à 60 % en poids de l'huile ou des huiles esters, de préférence de 5 à 50 % en poids.

### c. Huile non siliconée

La phase grasse liquide des compositions selon l'invention peut contenir en outre une ou plusieurs huiles non siliconées non volatiles.

On entend par « huile hydrocarbonée », une huile contenant principalement des atomes d'hydrogène et de carbone, et éventuellement des atomes d'oxygène, d'azote, de soufre et/ou de phosphore.

La phase grasse liquide peut contenir par exemple des huiles polaires telles que :
- les huiles végétales hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées ; ces huiles sont notamment les huiles de germe de blé, de maïs, de tournesol, de karité, de ricin, d'amandes douces, de macadamia, d'abricot, de soja, de colza, de coton, de luzerne, de pavot, de potimarron, de sésame, de courge, d'avocat, de noisette, de pépins de raisin ou de cassis, d'onagre, de millet, d'orge, de quinoa, d'olive, de seigle, de carthame, de bancoulier, de passiflore, de rosier muscat ; ou encore les triglycérides des acides caprylique/caprique comme ceux vendus par la société Stearines Dubois ou ceux vendus sous les dénominations Miglyol 810, 812 et 818 par la société Dynamit Nobel,
- les éthers de synthèse ayant de 10 à 40 atomes de carbone,
- les alcools gras en C₈ à C₂₆ comme l'alcool oléique ou l'octyldodécanol,
- les acides gras comme l'acide oléique, linoléique ou linolénique, et
- leurs mélanges.

La phase grasse liquide peut encore contenir des huiles apolaires telles que les hydrocarbures ou fluorocarbures, linéaires ou ramifiés, d'origine synthétique ou minérale, volatils ou non, comme les huiles de paraffine (telles que les isoparaffines) 1 et ses dérivés, la vaseline, les polydécènes, le polyisobutène hydrogéné tel que le parléam, le squalane, et leurs mélanges.

La ou les huiles non volatiles peuvent être présentes dans la composition selon l'invention en une teneur allant de 0,1 % à 90 % en poids, de préférence de 5 % à 80 % en poids par rapport au poids total de la composition.

Dans un mode de réalisation particulier, la composition exempte d'huile volatile.

### Actifs

La composition selon l'invention peut comprendre en outre au moins un actif. Par « actif », on entend un composé ayant un effet cosmétique et/ou dermatologique notamment sur les lèvres.

Cet actif peut être hydrophile ou hydrophobe. L'actif peut être hydrosoluble.

Ainsi, l'actif présent dans la composition selon l'invention peut être indépendamment choisi parmi :
- les agents dermorelaxants,
- les agents stimulant la synthèse de macromolécules dermiques ou épidermiques et/ou empêchant leur dégradation,
- les agents anti-glycation,
- les agents anti-irritants,
- les agents hydratants,
- les agents desquamants
- les agents modifiant la pigmentation,
- les inhibiteurs de la NO-synthase,
- les agents stimulant la prolifération des fibroblastes ou des kératinocytes et/ou la différentiation des kératinocytes,
- les agents anti-pollution ou anti-radicalaire,
- les agents apaisants,
- les agents agissant sur la microcirculation,
- les agents agissant sur le métabolisme énergétique des cellules,
- les agents cicatrisants, et
- leurs mélanges.

La quantité d'actif(s) va par exemple de 0,0001 à 30 % en poids et de préférence de 0,01 à 20 % en poids de matière active par rapport au poids total de la composition.

### Milieu Physiologiquement acceptable

La composition de l'invention doit être cosmétiquement ou dermatologiquement acceptable, à savoir contenir un milieu physiologiquement acceptable non toxique et susceptible d'être appliqué sur les lèvres d'êtres humains. Par cosmétiquement acceptable, on entend au sens de l'invention une composition d'aspect, d'odeur et de toucher agréables.

### Agent structurant

La composition selon l'invention peut comprendre, outre le ou les polyamide(s) siliconé(s) décrits plus haut, un agent structurant choisi parmi les cires, les polymères semi-cristallin, les gélifiants lipophiles et leur mélanges.

Il est entendu que la quantité en ces composés annexes peut être ajustée par l'homme du métier de manière à ne pas porter préjudice à l'effet recherché dans le cadre de la présente invention.

### Cire(s)

La cire considérée dans le cadre de la présente invention est d'une manière générale un composé lipophile, solide à température ambiante (25 °C), à changement d'état solide/liquide réversible, ayant un point de fusion supérieur ou égal à 30 °C pouvant aller jusqu'à 200 °C et notamment jusqu'à 120 °C.

En particulier, les cires convenant à l'invention peuvent présenter un point de fusion supérieur ou égal à 45 °C, et en particulier supérieur ou égal à 55 °C.

Au sens de l'invention, la température de fusion correspond à la température du pic le plus endothermique observé en analyse thermique (DSC) telle que décrite dans la norme ISO 11357-3 ; 1999. Le point de fusion de la cire peut être mesuré à l'aide d'un calorimètre à balayage différentiel (DSC), par exemple le calorimètre vendu sous la dénomination « MDSC 2920 » par la société TA Instruments.

Le protocole de mesure est le suivant :
Un échantillon de 5 mg de cire disposé dans un creuset est soumis à une première montée en température allant de -20 °C à 100 °C, à la vitesse de chauffe de 10 °C/minute, puis est refroidi de 100 °C à -20 °C à une vitesse de refroidissement de 10 °C/minute et enfin soumis à une deuxième montée en température allant de -20 °C à 100 °C à une vitesse de chauffe de 5 °C/minute. Pendant la deuxième montée en température, on mesure la variation de la différence de puissance absorbée par le creuset vide et par le creuset contenant l'échantillon de cire en fonction de la température. Le point de fusion du composé est la valeur de la température correspondant au sommet du pic de la courbe représentant la variation de la différence de puissance absorbée en fonction de la température.

Les cires susceptibles d'être utilisées dans les compositions selon l'invention sont choisies parmi les cires, solides, à température ambiante d'origine animale, végétale, minérale ou de synthèse et leurs mélanges.

A titre illustratif des cires convenant à l'invention, on peut notamment citer les cires hydrocarbonées comme la cire d'abeille, la cire de lanoline, et les cires d'insectes de Chine, la cire de son de riz, la cire de Carnauba, la cire de Candellila, la cire d'Ouricury, la cire d'Alfa, la cire de berry, la cire de shellac, la cire du Japon et la cire de sumac; la cire de montan, les cires d'orange et de citron, les cires microcristallines, les paraffines et l'ozokérite; les cires de polyéthylène, les cires obtenues par la synthèse de Fisher-Tropsch et les copolymères cireux ainsi que leurs esters.

On peut aussi citer des cires obtenues par hydrogénation catalytique d'huiles animales ou végétales ayant des chaînes grasses, linéaires ou ramifiées, en C₈-C₃₂, Parmi celles-ci, on peut notamment citer l'huile de jojoba isomérisée telle que l'huile de jojoba partiellement hydrogénée isomérisée trans fabriquée ou commercialisée par la société DESERT WHALE sous la référence commerciale Iso-Jojoba-50^{®} l'huile de tournesol hydrogénée, l'huile de ricin hydrogénée, l'huile de coprah hydrogénée, l'huile de lanoline hydrogénée, et le tétrastéarate de di-(triméthylol-1,1,1 propane) vendu sous la dénomination de Hest 2T-4S^{®} par la société HETERENE.

On peut encore citer les cires de silicone (C₃ₒ-₄ₛ ALKYL DIMETHICONE), les cires fluorées.

On peut également utiliser les cires obtenues par hydrogénation d'huile de ricin estérifiée avec l'alcool cétylique vendues sous les dénominations de Phytowax ricin 16L64^{®} et 22L73^{®} par la société SOPHIM. De telles cires sont décrites dans la demande FR-A- 2792190.

Comme cire, on peut utiliser un (hydroxystéaryloxy)stéarate d'alkyle en C₂₀-C₄₀ (le groupe alkyle comprenant de 20 à 40 atomes de carbone), seul ou en mélange.

Une telle cire est notamment vendue sous les dénominations « Kester Wax K 82 P^{®} », « Hydroxypolyester K 82 P^{®} » et « Kester Wax K 80 P^{®} » par la société KOSTER KEUNEN.

Comme micro cires pouvant être utilisées dans les compositions selon l'invention, on peut citer notamment les micro cires de carnauba telles que celle commercialisée sous la dénomination de MicroCare 350^{®} par la société MICRO POWDERS, les micro cires de cire synthétique telles que celle commercialisée sous la dénomination de MicroEase 114S^{®} par la société MICRO POWDERS, les micro cires constituées d'un mélange de cire de carnauba et de cire de polyéthylène telles que celles commercialisées sous les dénominations de Micro Care 300^{®} et 310^{®} par la société MICRO POWDERS, les micro cires constituées d'un mélange de cire de carnauba et de cire synthétique telles que celle commercialisée sous la dénomination Micro Care 325^{®} par la société MICRO POWDERS, les micro cires de polyéthylène telles que celles commercialisées sous les dénominations de Micropoly 200^{®}, 220^{®}, 220L^{®} et 250S^{®} par la société MICRO POWDERS et les micro cires de polytétrafluoroéthylène telles que celles commercialisées sous les dénominations de Microslip 519^{®} et 519 L^{®} par la société MICRO POWDERS.

La composition selon l'invention peut comprendre une teneur en cires allant de 0,1 à 30 % en poids par rapport au poids total de la composition, en particulier elle peut en contenir de 0,5 à 15 %, plus particulièrement de 1 à 10 %.

Selon un autre mode de réalisation, la composition selon l'invention est exempte de cire.

### Composés pâteux

La composition selon l'invention peut comprendre en outre un composé pâteux qui peut être avantageusement choisi parmi :
- la lanoline et ses dérivés
- les composés siliconés polymères ou non
- les composés fluorés polymères ou non
- les polymères vinyliques, notamment :
   - les homopolymères d'oléfines
   - les copolymères d'oléfines
   - les homopolymères et copolymères de diènes hydrogénés
   - les oligomères linéaires ou ramifiés, homo ou copolymères de (méth)acrylates d'alkyles ayant de préférence un groupement alkyle en C₈-C₃₀
   - les oligomères homo et copolymères d'esters vinyliques ayant des groupements alkyles en C₈-C₃₀
   - les oligomères homo et copolymères de vinyléthers ayant des groupements alkyles en C₈-C₃₀,
- les polyéthers liposolubles résultant de la polyéthérification entre un ou plusieurs diols en C₂-C₁₀₀, de préférence en C₂-C₅₀,
- les esters,
- et leurs mélanges.

Parmi les esters, on préfère notamment :
- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649 par la société Sasol,
- le propionate d'arachidyle commercialisé sous la marque Waxenol 801 par Alzo,
- les esters de phytostérol,
- les triglycérides d'acides gras et leurs dérivés,
- les esters de pentaérythritol,
- les polyesters non réticulés résultant de la polycondensation entre un acide dicarboxylique ou un polyacide carboxylique linéaire ou ramifié en C₄-C₅₀ et un diol ou un polyol en C₂-C₅₀,
- les esters aliphatiques d'ester résultant de l'estérification d'un ester d'acide hydroxycarboxylique aliphatique par un acide carboxylique aliphatique,
- les polyesters résultant de l'estérification, par un acide polycarboxylique, d'un ester d'acide hydroxy carboxylique aliphatique, ledit ester comprenant au moins deux groupes hydroxyle tels que les produits Risocast DA-H ^{®}, et Risocast DA-L ^{®},
- et leurs mélanges.

Parmi les composés pâteux d'origine végétale, on choisira de préférence un mélange de stérols de soja et de pentaérythritol oxyéthyléné (5OE) oxypropyléné (5 OP), commercialisé sous la référence Lanolide par la société VEVY.

Selon un mode de réalisation, la composition comprend moins de 10 % en poids, de préférence moins de 7 %, mieux moins de 5 %, et encore mieux moins de 3 % en poids de corps gras pâteux par rapport au poids total de la composition. De préférence encore, la composition est totalement exempte de corps gras pâteux.

### Résine Siliconée

La composition selon l'invention contient avantageusement au moins une résine siliconée. A titre d'exemple de ces résines silicones, on peut citer :
- les siloxysilicates qui peuvent être des triméthylsiloxysilicate de formule [(CH₃)₃SiO]ₓ(SiO_{4/2})_{y} (unités MQ) dans laquelle x et y sont des entiers allant de 50 à 80,
- les polysilesquioxanes de formule (CH₃SiO_{3/2})_{.x} (unités T) dans laquelle x est supérieur à 100 et dont au moins un des radicaux méthyle peut être substitué par un groupement R tel que défini plus haut,
- les polyméthylsilsesquioxanes qui sont des polysilsesquioxanes dans lesquels aucun des radicaux méthyle n'est substitué par un autre groupement. De tels polyméthylsilsesquioxanes sont décrits dans le document US 5,246,694 dont le contenu est incorporé par référence.

A titre d'exemples de résines polyméthylsilsesquioxanes commercialement disponibles, on peut citer celles qui sont commercialisés :
- par la société Wacker sous la référence Resin MK tels que la Belsil PMS MK : polymère comprenant des unités répétitives CH₃SiO_{3/2} (unités T), pouvant aussi comprendre jusqu'à 1% en poids d'unités (CH₃)₂SiO_{2/2} (unités D) et présentant un poids moléculaire moyen d'environ 10000,
- par la société SHIN-ETSU sous les références KR-220L qui sont composés d'unités T de formule CH₃SiO_{3/2} et ont des groupes terminaux Si-OH (silanol), sous la référence KR-242A qui comprennent 98% d'unités T et 2% d'unités diméthyle D et ont des groupes terminaux Si-OH ou encore sous la référence KR-251 comprenant 88% d'unités T et 12% d'unités dimethyl D et ont des groupes terminaux Si-OH.

Comme résines siloxysilicates, on peut citer les résines triméthylsiloxysilicate (TMS) éventuellement sous forme de poudres. De telles résines sont commercialisées sous la référence SR1000 par la société General Electric ou sous la référence TMS 803 par la société Wacker. On peut encore citer les résines timéthylsiloxysilicate commercialisées dans un solvant tel que la cyclométhicone, vendues sous la dénomination "KF-7312J" par la société Shin-Etsu, "DC 749", "DC 593" par la société Dow Corning.

Avantageusement, la résine siliconée, telle que la résine triméthylsiloxysilicates par exemple, est présente dans une teneur allant de 0,5 à 30% par rapport au poids total de la composition, ou mieux de 1 à 25% ou encore mieux, de 5 à 25%.

De préférence la résine siliconée, et notamment la résine triméthylsiloxysilicates, est présente dans un ratio tel que la proportion massique polyamide siliconé/ résine siliconée est comprise entre 1/₄ et 4, et de préférence entre 1/3 et 3.

### Gélifiants lipophiles

Les gélifiants utilisables dans les compositions selon l'invention peuvent être des gélifiants lipophiles organiques ou minéraux, polymériques ou moléculaires.

Comme gélifiant lipophile minéral, on peut citer les argiles éventuellement modifiées comme les hectorites modifiées par un chlorure d'ammonium en C₁₀ à C₂₂, comme l'hectorite modifiée par du chlorure de di-stéaryl di-méthyl ammonium telle que, par exemple, celle commercialisée sous la dénomination de Bentone 38V^{®} par la société ELEMENTIS.

On peut également citer la silice pyrogénée éventuellement traitée hydrophobe en surface dont la taille des particules est inférieure à 1 µm. Il est en effet possible de modifier chimiquement la surface de la silice, par réaction chimique générant une diminution du nombre de groupes silanol présents à la surface de la silice. On peut notamment substituer des groupes silanol par des groupements hydrophobes : on obtient alors une silice hydrophobe. Les groupements hydrophobes peuvent être :
- des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (8ème édition, 2000). Elles sont par exemple commercialisées sous les références Aerosil R812^{®} par la société DEGUSSA, CAB-O-SIL TS-530^{®} par la société CABOT,
- des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées « Silica diméthyl silylate » selon le CTFA (8ème édition, 2000). Elles sont par exemple commercialisées sous les références Aerosil R972^{®}, et Aerosil R974^{®} par la société DEGUSSA, CAB-O-SIL TS-610^{®} et CAB-O-SIL TS-720^{®} par la société CABOT.

La silice pyrogénée hydrophobe présente en particulier une taille de particules pouvant être nanométrique à micrométrique, par exemple allant d'environ de 5 à 200 nm.

Les gélifiants lipophiles organiques polymériques sont par exemple les organopolysiloxanes élastomériques partiellement ou totalement réticulés, de structure tridimensionnelle, comme ceux commercialisés sous les dénominations de KSG6^{®}, KSG16^{®} et de KSG18^{®} par la société SHIN-ETSU, de Trefil E-505C^{®} et Trefil E-506C^{®} par la société DOW-CORNING, de Gransil SR-CYC^{®}, SR DMF10^{®}, SR-DC556^{®}, SR 5CYC gel^{®}, SR DMF 10 gel^{®} et de SR DC 556 gel^{®} par la société GRANT INDUSTRIES, de SF 1204^{®} et de JK 113^{®} par la société GENERAL ELECTRIC ; l'éthylcellulose comme celle vendue sous la dénomination Ethocel^{®} par la société DOW CHEMICAL ; les galactommananes comportant de un à six, et en particulier de deux à quatre, groupes hydroxyle par ose, substitués par une chaîne alkyle saturée ou non, comme la gomme de guar alkylée par des chaînes alkyle en C₁ à C₆, et en particulier en C₁ à C₃ et leurs mélanges. Les copolymères séquencés de type « dibloc », « tribloc » ou « radial » du type polystyrène/polyisoprène, polystyrène/polybutadiène tels que ceux commercialisés sous la dénomination Luvitol HSB^{®} par la société BASF, du type polystyrène/copoly(éthylène-propylène) tels que ceux commercialisés sous la dénomination de Kraton^{®} par la société SHELL CHEMICAL CO ou encore du type polystyrène/copoly(éthylène-butylène), les mélanges de copolymères tribloc et radial (en étoile) dans l'isododécane tels que ceux commercialisé par la société PENRECO sous la dénomination Versagel^{®} comme par exemple le mélange de copolymère tribloc butylène/éthylène/styrène et de copolymère étoile éthylène/propylène/styrène dans l'isododécane (Versagel M 5960).

Parmi les gélifiants lipophiles pouvant être utilisés dans les compositions selon l'invention, on peut encore citer les esters de dextrine et d'acide gras, tels que les palmitates de dextrine, notamment tels que ceux commercialisés sous les dénominations Rheopearl TL^{®} ou Rheopearl KL^{®} par la société CHIBA FLOUR.

### Phase aqueuse

Selon un premier mode de réalisation de l'invention, la composition selon l'invention peut comprendre un milieu aqueux, constituant une phase aqueuse, qui peut former la phase dispersée de la composition.

La phase aqueuse peut être constituée essentiellement d'eau ; elle peut également comprendre un mélange d'eau et de solvant miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, les aldéhydes en C₂-C₄ et leur mélanges.

La phase aqueuse (eau et éventuellement le solvant miscible à l'eau) peut être présente, en une teneur allant de 1 % à 95 % en poids, par rapport au poids total de la composition, de préférence allant de 2 % à 80 % en poids, et préférentiellement allant de 3 % à 60 % en poids.

La composition selon l'invention peut également contenir moins de 10 % en poids voire moins de 4 % en poids de phase aqueuse ou d'eau, voire est totalement anhydre.

### Matières colorantes

Selon un mode de réalisation, la composition selon l'invention peut contenir en outre au moins un agent de coloration qui peut être choisi parmi les colorants hydrosolubles ou liposolubles, les pigments, les nacres et leurs mélanges.

La composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules blanches ou colorées, minérales ou organiques, insolubles dans une solution aqueuse, destinées à colorer et/ou opacifier le film résultant.

Les pigments peuvent être présents à raison de 0,01 à 20 % en poids, notamment de 0,01 à 15 % en poids, et en particulier de 0,02 à 10 % en poids, par rapport au poids total de la composition cosmétique.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome.

Il peut également s'agir de pigment ayant une structure qui peut être par exemple de type séricite/oxyde de fer brun/dioxyde de titane/silice. Un tel pigment est commercialisé par exemple sous la référence COVERLEAF NS ou JS par la société CHEMICALS AND CATALYSTS et présente un rapport de contraste voisin de 30.

La matière colorante peut encore comporter un pigment ayant une structure qui peut être par exemple de type microsphères de silice contenant de l'oxyde de fer. Un exemple de pigment présentant cette structure est celui commercialisé par la société MIYOSHI sous la référence PC BALL PC-LL-100 P, ce pigment étant constitué de microsphères de silice contenant de l'oxyde de fer jaune.

Parmi les pigments organiques utilisables dans l'invention, on peut citer le noir de carbone, les pigments de type D & C, les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Par « nacres », il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica titane recouvert d' oxychlorure de bismuth, le mica titane recouvert avec de l'oxyde de chrome, le mica titane recouvert avec un colorant organique ainsi que les pigments nacrés à base d' oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d' oxychlorure de bismuth.

Parmi les nacres disponibles sur le marché, on peut citer les nacres TIMICA, FLAMENCO et DUOCHROME (sur base de mica) commercialisées par la société ENGELHARD, les nacres TIMIRON commercialisées par la société MERCK, les nacres sur base de mica PRESTIGE commercialisées par la société ECKART et les nacres sur base de mica synthétique SUNSHINE commercialisées par la société SUN CHEMICAL.

Les nacres peuvent plus particulièrement posséder une couleur ou un reflet jaune, rose, rouge, bronze, orangé, brun, or et/ou cuivré.

A titre illustratif des nacres pouvant être mises en oeuvre dans le cadre de la présente invention, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

Par « colorants », il faut comprendre des composés généralement organiques solubles dans les corps gras comme les huiles ou dans une phase hydroalcoolique.

La composition cosmétique selon l'invention peut comprendre également des colorants hydrosolubles ou liposolubles. Les colorants liposolubles sont par exemple le rouge Soudan, le DC Red 17, le DC Green 6, le β-carotène, le brun Soudan, le DC Yellow 11, le DC Violet 2, le DC orange 5, le jaune quinoléine. Les colorants hydrosolubles sont par exemple le jus de betterave, le bleu de méthylène.

La composition cosmétique selon l'invention peut également contenir au moins un matériau à effet optique spécifique.

Cet effet est différent d'un simple effet de teinte conventionnel, c'est-à-dire unifié et stabilisé tel que produit par les matières colorantes classiques comme par exemple les pigments monochromatiques. Au sens de l'invention, « stabilisé » signifie dénué d'effet de variabilité de la couleur avec l'angle d'observation ou encore en réponse à un changement de température.

Par exemple, ce matériau peut être choisi parmi les particules à reflet métallique, les agents de coloration goniochromatiques, les pigments diffractants, les agents thermochromes, les agents azurants optiques, ainsi que les fibres, notamment interférentielles. Bien entendu, ces différents matériaux peuvent être associés de manière à procurer la manifestation simultanée de deux effets, voire d'un nouvel effet conforme à l'invention.

Les particules à reflet métallique utilisables dans l'invention sont en particulier choisies parmi :
- les particules d'au moins un métal et/ou d'au moins un dérivé métallique,
- les particules comportant un substrat, organique ou minéral, monomatière ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple les bronzes et les laitons) sont des métaux préférés.

Par « dérivés métalliques », on désigne des composés dérivés de métaux notamment des oxydes, des fluorures, des chlorures et des sulfures

A titre illustratif de ces particules, on peut citer des particules d'aluminium, telles que celles commercialisées sous les dénominations STARBRITE 1200 EAC^{®} par la société SIBERLINE et METALURE^{®} par la société ECKART.

On peut également citer les poudres métalliques de cuivre ou des mélanges d'alliage telles les références 2844 commercialisées par la société RADIUM BRONZE, les pigments métalliques comme l'aluminium ou le bronze, telles que celles commercialisées sous les dénominations ROTOSAFE 700 de la société ECKART, les particules d'aluminium enrobé de silice commercialisées sous la dénomination VISIONAIRE BRIGHT SILVER de la société ECKART et les particules d'alliage métallique comme des poudres de bronze (alliage cuivre et zinc) enrobé de silice commercialisées sous la dénomination de Visionaire Bright Natural Gold de la société Eckart.

Il peut encore s'agir de particules comportant un substrat de verre comme celles commercialisées par la société NIPPON SHEET GLASS sous les dénominations MICROGLASS METASHINE.

L'agent de coloration goniochromatique peut être choisi par exemple parmi les structures multicouches interférentielles et les agents de coloration à cristaux liquides.

Des exemples de structures multicouche interférentielles symétriques utilisables dans des compositions réalisées conformément à l'invention sont par exemple les structures suivantes : Al/SiO₂/Al/SiO₂/Al, des pigments ayant cette structure étant commercialisés par la société DUPONT DE NEMOURS ; Cr/MgF₂/Al/MgF₂/Cr, des pigments ayant cette structure étant commercialisés sous la dénomination CHROMAFLAIR par la société FLEX ; MOS₂/SiO₂/Al/SiO₂/MOS₂ ; Fe₂O₃/SiO₂/Al/SiO₂/Fe₂O₃, et Fe₂O₃/SiO₂/Fe₂O₃/SiO₂/Fe₂O₃, des pigments ayant ces structures étant commercialisés sous la dénomination SICOPEARL par la société BASF ; MoS₂/SiO₂/mica-oxyde/SiO₂MoS₂ ; Fe₂O₃/SiO₂/mica-oxyde/SiO₂/Fe₂O₃ ; TiO₂/SiO₂/TiO₂ et TiO₂/Al₂O₃/TiO₂ ; SnO/TiO₂/SiO₂/TiO₂/SnO ; Fe₂O₃/SiO₂/Fe₂O₃; SnO/mica/TiO₂/SiO₂/TiO₂/mica/SnO, des pigments ayant ces structures étant commercialisés sous la dénomination XIRONA par la société MERCK (Darmstadt). A titre d'exemple, ces pigments peuvent être les pigments de structure silice/oxyde de titane/oxyde d'étain commercialisés sous le nom XIRONA MAGIC par la société MERCK, les pigments de structure silice/oxyde de fer brun commercialisés sous le nom XIRONA INDIAN SUMMER par la société MERCK et les pigments de structure silice/oxyde de titane/mica/oxyde d'étain commercialisés sous le nom XIRONA CARRIBEAN BLUE par la société MERCK. On peut encore citer les pigments INFINITE COLORS de la société SHISEIDO. Selon l'épaisseur et la nature des différentes couches, on obtient différents effets. Ainsi, avec la structure Fe₂O₃/SiO₂/Al/ SiO₂/Fe₂O₃ on passe du doré-vert au gris-rouge pour des couches de SiO₂ de 320 à 350 nm ; du rouge au doré pour des couches de SiO₂ de 380 à 400 nm ; du violet au vert pour des couches de SiO₂ de 410 à 420 nm ; du cuivre au rouge pour des couches de SiO₂ de 430 à 440 nm.

On peut citer, à titre d'exemple de pigments à structure multicouche polymérique, ceux commercialisés par la société 3M sous la dénomination COLOR GLITTER.

Comme particules goniochromatiques à cristaux liquides, on peut utiliser par exemple celles vendues par la société CHENIX ainsi que celle commercialisées sous la dénomination HELICONE^{®} HC par la société WACKER.

### Charge

La composition selon l'invention peut comprendre au moins une charge, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon^{®}) (Orgasol^{®} de chez Atochem), de poly-β-alanine et de polyéthylène, les poudres de polymères de tétrafluoroéthylène (Téflon^{®}), la lauroyllysine, l'amidon, le nitrure de bore, les microsphères creuses polymériques telles que celles de chlorure de polyvinylidène/acrylonitrile comme l'Expancel^{®} (Nobel Industrie), de copolymères d'acide acrylique (Polytrap^{®} de la Société Dow Corning) et les microbilles de résine de silicone (Tospearls^{®} de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads^{®} de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

### Ingrédients cosmétiques usuels additionnels

La composition selon l'invention peut comprendre en outre tout ingrédient cosmétique usuel pouvant être choisi notamment parmi les antioxydants, les parfums, les conservateurs, les neutralisants, les tensioactifs, les filtres solaires, les vitamines, les hydratants, les composés auto-bronzants, les actifs antirides, les émollients, les actifs hydrophiles ou lipophiles, les agents anti-radicaux libres, les agents déodorants, les sequestrants, les agents filmogènes, et leurs mélanges.

Bien entendu, l'homme du métier veillera à choisir les éventuels ingrédients complémentaires et/ou leur quantité de telle manière que les propriétés avantageuses de la composition selon l'invention ne soient pas ou substantiellement pas altérées par l'adjonction envisagée.

Le produit selon l'invention peut être avantageusement utilisé pour le maquillage de la peau et/ou des lèvres et/ou des phanères selon la nature des ingrédients utilisés. En particulier, le produit de l'invention peut se présenter sous forme de fond de teint solide, de bâton ou pâte de rouge à lèvres, de produit anti-cernes, ou contours des yeux, d'eye liner, de mascara, d'ombre à paupières, de produit de maquillage du corps ou encore un produit de coloration de la peau.

Les compositions du produit de l'invention peuvent être obtenues par chauffage des différents constituants à une température correspondant à la température de fusion du ou des ingrédients la plus élevée, puis coulage du mélange fondu dans un moule (coupelle ou doigt de gant). Elles peuvent aussi être obtenues par extrusion comme décrit dans la demande EP-A-0 667 146.

En particulier, la composition de l'invention peut se présenter sous la forme d'un produit coloré de maquillage des lèvres comme un rouge à lèvres, un brillant à lèvres ou un crayon, présentant éventuellement des propriétés de soin ou de traitement. Elle peut se présenter sous la forme d'un stick anhydre.

La composition selon l'invention peut être fabriquée par les procédés connus, généralement utilisés dans le domaine cosmétique ou dermatologique. Par exemple, elle peut être fabriquée par le procédé suivant :
Dans un premier temps, les charges et les pigments peuvent être broyés dans une partie de la phase huileuse.

Le reste des ingrédients liposolubles peuvent être ensuite mélangés à une température de l'ordre de 100 °C. Le broyat ou les actifs pré-dispersés peuvent alors être ajoutés dans la phase huileuse.

Les éventuels actifs hydrophiles peuvent ensuite être dispersés à l'aide d'un agitateur mécanique.

Enfin, la composition peut être coulée dans un moule apte à lui procurer la forme de dôme et le tout peut être laissé refroidir à température ambiante.

Selon un autre aspect, l'invention concerne également un ensemble cosmétique comprenant :
i) un récipient délimitant un compartiment, ledit récipient étant fermé par un élément de fermeture ; et
ii) une composition conforme à l'invention disposée à l'intérieur dudit compartiment.

Le récipient peut être sous toute forme adéquate. Il peut être notamment sous forme d'un pot, d'un étui, d'une boite, ou d'un boîtier.

L'élément de fermeture peut être sous forme d'un bouchon amovible, d'un couvercle, d'un opercule, d'une bande déchirable, ou d'une capsule, notamment du type comportant un corps fixé au récipient et une casquette articulée sur le corps. Il peut être également sous forme d'un élément assurant la fermeture sélective du récipient, notamment une pompe, une valve, tel qu'un clapet par exemple.

Les exemples suivants sont donnés à titre illustratif et non limitatif de la présente invention. Les pourcentages sont des pourcentages en poids.

### Exemples 1 et 2 de rouges à lèvres

Les compositions des exemples 1 et 2 sont obtenues selon le protocole suivant.

Dans un premier temps, les charges et les pigments sont broyés dans une partie de la phase huileuse.

Le reste des ingrédients liposolubles et les tensioactifs (cetyl PEG/PGG-10/1 diméthicone et phosphate de trioleyle) sont ensuite mélangés à une température de l'ordre de 100 °C. Le broyat ou les actifs pré-dispersés sont alors ajoutés dans la phase huileuse.

Les actifs hydrophiles sont ensuite dispersés à l'aide d'un agitateur Moritz.

Enfin, la composition est coulée dans un moule pour lui donner la forme de dôme et le tout est laissé refroidir à température ambiante.

| **Composé (nom INCI US)** | **Exemple 1 (comparatif)** | **Exemple 2 (Invention)** |
|---|---|---|
| LAUROYL LYSINE | 2 | 2 |
| SODIUM SACCHARIN | 0,02 | 0,02 |
| OXYDES DE FER BRUN,JAUNE (75/25) (CI: 77491 + 77492) | 0,02 | 0,02 |
| TITANIUM DIOXIDE | 0,50 | 0,50 |
| RED 28 LAKE | 0,0040 | 0,0040 |
| FRAGRANCE | 0,30 | 0,30 |
| HYDROGENATED POLYISOBUTENE | 15,11 | 13,11 |
| HYDROGENATED STYRENE/METHYL STYRENE/INDENE COPOLYMER (REGALITE R 1100) | - | 2 |
| DIMETHICONE (VISCOSITE: 10 CST) | 12,66 | 12,66 |
| PHENYL TRIMETHICONE (VISCOSITE: 20 CST - PM: 372) | 36,306 | 36,306 |
| CETYL PEG/PPG-10/1 DIMETHICONE (20/75/5 - VISCOSITE: 3000 CST) | 1 | 1 |
| NYLON-611/DIMETHICONE COPOLYMER (=polyamide/polydiméthyl siloxane DC 2-8179 de Dow Corning ) (m>50) | 22,50 | 22,50 |
| NYLON-611/DIMETHICONE COPOLYMER (and) PPG-3 MYRISTYL ETHER (=polyamide/polydiméthyl siloxane DC 2-8178 de Dow Corning ) (m<50) | 1,08 | 1,08 |
| GLYCERINE | 7,50 | 7,50 |
| TRIOLEYL PHOSPHATE | 1 | 1 |
| Total | 100 | 100 |

On obtient pour l'exemple comparatif 1 une composition présentant :
- une dureté de 156g, et
- une élasticité de 93% .

On obtient pour l'exemple 2 selon l'invention une composition présentant :
- une dureté de 164g, et
- une élasticité de 93%.

La composition selon l'invention présente une dureté améliorée sans pour autant perdre en élasticité par rapport à l'exemple comparatif. La composition selon l'invention présente ainsi une résistance améliorée et risque moins de se fracturer lors de son utilisation.

De plus, on constate que la composition selon l'invention de l'exemple 2 présente des qualités d'application et un résultat de maquillage améliorés par rapport à l'exemple comparatif 1, notamment en terme de brillance et de glissant à l'application tout en conservant une dureté et une élasticité comparable ou supérieure à celle de l'exemple 1.

Par ailleurs, on observe également une amélioration de la stabilité de la composition selon l'invention par rapport à celle de l'exemple comparatif, en particulier de la non exsudation.

## Revendications

1. Composition cosmétique solide de maquillage ou de soin comprenant dans un milieu physiologiquement acceptable au moins une phase grasse liquide comprenant :
- au moins une résine hydrocarbonée indénique, de poids moléculaire moyen en nombre inférieur ou égal à 10000 g/mol,
- au moins un polyamide siliconé,
ledit polyamide siliconé comprenant au moins une portion siliconée présentant un degré de polymérisation moyen supérieur ou égal à 50 et représentant au moins 10% du poids total de ladite composition
- au moins une huile,
ladite composition étant exempte d'huile volatile.

2. Composition selon la revendication précédente, **caractérisé en ce que** ledit polyamide siliconé représente au moins 15%, ou mieux au moins 20%, par rapport au poids total de ladite composition.

3. Composition selon l'une quelconque des revendications précédentes, comprenant moins de 10 %, voire moins de 4 % d'eau, en particulier étant anhydre.

4. Composition selon l'une quelconque des revendications précédentes, dans laquelle le polyamide siliconé comprend au moins un motif répondant à la formule générale I : 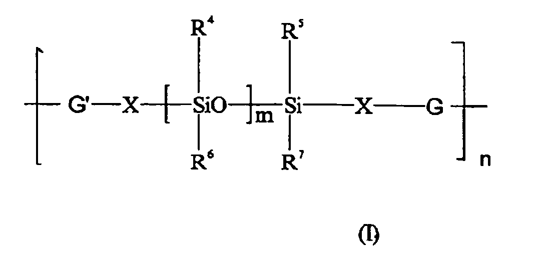
1) dans laquelle : G' représente C(O) quand G représente -C(O)-NH-Y-NH-, et G' représente -NH- quand G représente NH-C(O)-Y-C(O)-
2) R⁴, R⁵, R⁶ et R⁷, identiques ou différents, représentent un groupe choisi parmi :
- les groupes hydrocarbonés, linéaires, ramifiés ou cycliques, en C₁ à C₄₀, saturés ou insaturés, pouvant contenir dans leur chaîne un ou plusieurs atomes d'oxygène, de soufre et/ou d'azote, et pouvant être substitués en partie ou totalement par des atomes de fluor,
- les groupes aryles en C₆ à C₁₀, éventuellement substitués par un ou plusieurs groupes alkyle en C₁ à C₄,
- les chaînes polyorganosiloxanes contenant ou non un ou plusieurs atomes d'oxygène, de soufre et/ou d'azote,
3) les X, identiques ou différents, représentent un groupe alkylène di-yle, linéaire ou ramifié en C₁ à C₃₀, pouvant contenir dans sa chaîne un ou plusieurs atomes d'oxygène et/ou d'azote,
4) Y est un groupe divalent alkylène linéaire ou ramifié, arylène, cycloalkylène, alkylarylène ou arylalkylène, saturé ou insaturé, en C₁ à C₅₀, pouvant comporter un ou plusieurs atomes d'oxygène, de soufre et/ou d'azote, et/ou porter comme substituant l'un des atomes ou groupes d'atomes suivants : fluor, hydroxy, cycloalkyle en C₃ à C₈, alkyle en C₁ à C₄₀, aryle en C₅ à C₁₀, phényle éventuellement substitué par 1 à 3 groupes alkyle en C₁ à C₃, hydroxyalkyle en C₁ à C₃ et amino alkyle en C₁ à C₆, ou
5) Y représente un groupe répondant à la formule : 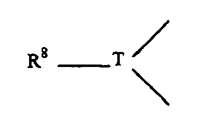 dans laquelle
- T représente un groupe hydrocarboné trivalent ou tétravalent, linéaire ou ramifié, saturé ou insaturé, en C₃ à C₂₄ éventuellement substitué par une chaîne polyorganosiloxane, et pouvant contenir un ou plusieurs atomes choisis parmi O, N et S, ou T représente un atome trivalent choisi parmi N, P et Al, et
- R⁸ représente un groupe alkyle en C₁ à C₅₀, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,
6) n est un nombre entier allant de 2 à 500, de préférence de 2 à 200, et m est un nombre entier allant de 50 à 1000, de préférence de 50 à 700 et mieux encore de 50 à 200, « m » correspondant au degré de polymérisation moyen de la portion siliconée du polyamide siliconé.

5. Composition selon la revendication précédente, dans laquelle X et/ou Y représentent un groupe alkylène contenant dans sa partie alkylène au moins l'un des éléments suivants :
1) 1 à 5 groupes amides, urée, uréthane, ou carbamate,
2) un groupe cycloalkyle en C₅ ou C₆, et
3) un groupe phénylène éventuellement substitué par 1 à 3 groupes alkyles identiques ou différents en C₁ à C₃,
éventuellement substitué par au moins un élément choisi dans le groupe constitué de :
- un groupe hydroxy,
- un groupe cycloalkyle en C₃ à C₈,
- un à trois groupes alkyles en C₁ à C₄₀,
- un groupe phényle éventuellement substitué par un à trois groupes alkyles en C₁ à C₃,
- un groupe hydroxyalkyle en C₁ à C₃, et
- un groupe aminoalkyle en C₁ à C₆.

6. Composition selon l'une quelconque des revendications 5 et 6, dans laquelle R⁴, R⁵, R⁶ et R⁷ représentent, indépendamment, un groupe alkyle en C₁ à C₄₀, linéaire ou ramifié, de préférence un groupe CH₃, C₂H₅, n-C₃H₇ ou isopropyle, une chaîne polyorganosiloxane ou un groupe phényle éventuellement substitué par un à trois groupes méthyle ou éthyle.

7. Composition selon l'une quelconque des revendications précédentes, comprenant :
- au moins un premier polyamide siliconé comprenant au moins un motif de formule (I) telle que définie en revendication 5 dans laquelle m varie de 50 à 600, en particulier de 60 à 400, notamment de 75 à 200 et plus particulièrement est de l'ordre de 120 pour le premier polymère, et
- au moins un second polyamide siliconé comprenant au moins un second motif de formule (I) telle que définie en revendication 5 dans laquelle G', G, R⁴, R⁵, R⁶, R⁷, X, Y et n sont tels que définis dans la revendication 5 et m va de 5 à 100, en particulier de 10 à 75 et plus particulièrement est de l'ordre de 15 pour le second polymère.

8. Composition selon la revendication précédente, **caractérisée en ce que** la résine hydrocarbonée indénique est hydrogénée.

9. Composition selon l'une quelconque des revendications 1 à 8, **caractérisée en ce que** la résine est une résine indénique choisie parmi les copolymères indène/méthylstyrène/styrène hydrogénés.

10. Composition selon l'une quelconque des revendications précédentes, **caractérisée en ce que** la résine est présente en une teneur allant de 0,1 à 30 % en poids, par rapport au poids total de la composition, de préférence allant de 0,3 à 20 % en poids, plus préférentiellement allant de 0,5 à 15 % en poids.

11. Composition selon l'une quelconque des revendications précédentes, **caractérisée en ce que** le ratio massique polyamide siliconé/ résine va de 2 à 40.

12. Composition selon l'une quelconque des revendications précédentes, **caractérisée en ce qu'**elle comprend au moins une huile siliconée, une huile ester ou une huile non siliconée.

13. Composition selon l'une des revendications précédentes, **caractérisée en ce qu'**elle présente une élasticité supérieure à 80%.

14. Procédé de maquillage de la peau et/ou des lèvres et/ou des phanères, dans lequel on applique une composition telle que définie selon l'une quelconque des revendications précédentes.

## Claims

1. Solid makeup or care cosmetic composition comprising, in a physiologically acceptable medium, at least one liquid fatty phase comprising:
- at least one indene hydrocarbon-based resin with a number-average molecular weight of less than or equal to 10 000 g/mol,
- at least one silicone polyamide,
the said silicone polyamide comprising at least one silicone portion with a mean degree of polymerization of greater than or equal to 50 and representing at least 10% of the total weight of the said composition
- at least one oil,
- said composition being free of volatile oil.

2. Composition according to the preceding claim, **characterized in that** the said silicone polyamide represents at least 15% and better still at least 20% relative to the total weight of the said composition.

3. Composition according to either of the preceding claims, comprising less than 10% or even less than 4% of water, in particular being anhydrous.

4. Composition according to any one of the preceding claims, in which the silicone polyamide comprises at least one unit corresponding to the general formula I: 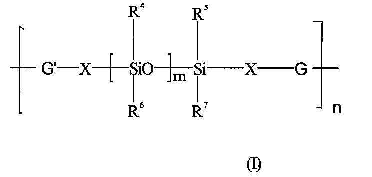 in which:
1) G' represents C(O) when G represents -C(O)-NH-Y-NH-, and G' represents -NH- when G represents -NH-C(O)-Y-C(O)-,
2) R⁴, R⁵, R⁶ and R⁷, which may be identical or different, represent a group chosen from:
- linear, branched or cyclic, saturated or unsaturated, C₁-C₄₀ hydrocarbon-based groups, possibly containing in their chain one or more oxygen, sulfur and/or nitrogen atoms, and possibly being partially or totally substituted with fluorine atoms,
- C₆-C₁₀ aryl groups, optionally substituted with one or more C₁-C₄ alkyl groups,
- polyorganosiloxane chains possibly containing one or more oxygen, sulfur and/or nitrogen atoms;
3) the groups X, which may be identical or different, represent a linear or branched C₁-C₃₀ alkylenediyl group, possibly containing in its chain one or more oxygen and/or nitrogen atoms;
4) Y is a saturated or unsaturated, C₁-C₅₀ linear or branched divalent alkylene, arylene, cycloalkylene, alkylarylene or arylalkylene group, possibly comprising one or more oxygen, sulfur and/or nitrogen atoms, and/or bearing as substituent one of the following atoms or groups of atoms: fluorine, hydroxyl, C₃-C₈ cycloalkyl, C₁-C₄₀ alkyl, C₅-C₁₀ aryl, phenyl optionally substituted with 1 to 3 C₁-C₃ alkyl, C₁-C₃ hydroxyalkyl and C₁-C₆ aminoalkyl groups; or
5) Y represents a group corresponding to the formula: 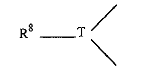 in which
- T represents a linear or branched, saturated or unsaturated, C₃-C₂₄ trivalent or tetravalent hydrocarbon-based group optionally substituted with a polyorganosiloxane chain, and possibly containing one or more atoms chosen from O, N and S, or T represents a trivalent atom chosen from N, P and Al, and
- R⁸ represents a linear or branched C₁-C₅₀ alkyl group or a polyorganosiloxane chain, possibly comprising one or more ester, amide, urethane, thiocarbamate, urea, thiourea and/or sulfonamide groups, which may possibly be linked to another chain of the polymer;
6) n is an integer ranging from 2 to 500 and preferably from 2 to 200, and m is an integer ranging from 50 to 1000, preferably from 50 to 700 and better still from 50 to 200, "m" corresponding to the mean degree of polymerization of the silicone portion of the silicone polyamide.

5. Composition according to the preceding claim, in which X and/or Y represent(s) an alkylene group containing in its alkylene portion at least one of the following components:
1) 1 to 5 amide, urea, urethane or carbamate groups,
2) a C₅ or C₆ cycloalkyl group, and
3) a phenylene group optionally substituted with 1 to 3 identical or different C₁-C₃ alkyl groups, optionally substituted with at least one component chosen from the group consisting of:
- a hydroxyl group,
- a C₃-C₈ cycloalkyl group,
- one to three C₁-C₄₀ alkyl groups,
- a phenyl group optionally substituted with one to three C₁-C₃ alkyl groups,
- a C₁-C₃ hydroxyalkyl group, and
- a C₁-C₆ aminoalkyl group.

6. Composition according to either of Claims 4 and 5, in which R⁴, R⁵, R⁶ and R⁷ independently represent a linear or branched C₁-C₄₀ alkyl group, preferably a CH₃, C₂H₅, n-C₃H₇ or isopropyl group, a polyorganosiloxane chain or a phenyl group optionally substituted with one to three methyl or ethyl groups.

7. Composition according to any one of the preceding claims, comprising:
- at least one first silicone polyamide comprising at least one unit of formula (I) as defined in Claim 5, in which m ranges from 50 to 600, in particular from 60 to 400 and especially from 75 to 200, and is more particularly about 120 for the first polymer, and
- at least one second silicone polyamide comprising at least one second unit of formula (I) as defined in Claim 5, in which G', G, R⁴, R⁵, R⁶, R⁷, X, Y and n are as defined in Claim 5 and m ranges from 5 to 100, in particular from 10 to 75, and is more particularly about 15 for the second polymer.

8. Composition according to the preceding claim, **characterized in that** the indene hydrocarbon-based resin is hydrogenated.

9. Composition according to any one of Claims 1 to 8, **characterized in that** the resin is an indene resin chosen from hydrogenated indene/methylstyrene/styrene copolymers.

10. Composition according to any one of the preceding claims, **characterized in that** the resin is present in a content ranging from 0.1% to 30% by weight, preferably ranging from 0.3% to 20% by weight and more preferentially ranging from 0.5% to 15% by weight relative to the total weight of the composition.

11. Composition according to any one of the preceding claims, **characterized in that** the silicone polyamide/resin mass ratio ranges from 2 to 40.

12. Composition according to any one of the preceding claims, **characterized in that** it comprises at least one silicone oil, an ester oil or a non-silicone oil.

13. Composition according to any one of the preceding claims, **characterised in that** it has an elasticity of greater than 80%.

14. Process for making up the skin and/or the lips and/or the integuments, in which a composition as defined according to any one of the preceding claims is applied.

## Patentansprüche

1. Feste kosmetische Schmink- oder Pflegezusammensetzung, umfassend in einem physiologisch unbedenklichen Medium eine flüssige Fettphase, umfassend:
- mindestens ein Inden-Kohlenwasserstoffharz mit einem zahlenmittleren Molekulargewicht kleiner gleich 10.000 g/mol,
- mindestens ein Silikonpolyamid,
wobei das Silikonpolyamid mindestens einen Silikonteil mit einem durchschnittlichen Polymerisationsgrad größer gleich 50 aufweist und mindestens 10 Gew.-% des Gesamtgewichts der Zusammensetzung ausmacht,
- mindestens ein Öl,
wobei die Zusammensetzung von flüchtigem Öl frei ist.

2. Zusammensetzung nach dem vorhergehenden Anspruch, **dadurch gekennzeichnet, dass** das Silikonpolyamid mindestens 15 Gew.-% oder besser mindestens 20 Gew.-%, bezogen auf das Gesamtgewicht der Zusammensetzung, ausmacht.

3. Zusammensetzung nach einem der vorhergehenden Ansprüche, die weniger als 10% oder sogar weniger als 4% Wasser umfasst und insbesondere wasserfrei ist.

4. Zusammensetzung nach einem der vorhergehenden Ansprüche, wobei das Silikonpolyamid mindestens eine Einheit der allgemeinen Formel I umfasst: 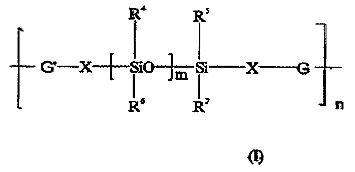
1) in der: G' für C(O) steht, wenn G für -C(O)-NH-Y-NH- steht, und G' für -NH- steht, wenn G für -NH-C(O)-Y-NH- steht,
2) R⁴, R⁵, R⁶ und R⁷ gleich oder verschieden sind und für eine aus
- linearen, verzweigten oder cyclischen, gesättigten oder ungesättigten C₁- bis C₄₀-Kohlenwasserstoffgruppen, die in ihrer Kette ein oder mehrere Sauerstoff-, Schwefel- und/oder Stickstoffatome enthalten können und teilweise oder vollständig durch Fluoratome substituiert sein können,
- C₆- bis C₁₀-Arylgruppen, die gegebenenfalls durch eine oder mherere C₁- bis C₄-Alkylgruppen substituiert sind,
- Polyorganosiloxanketten, die gegebenenfalls ein oder mehrere Sauerstoff-, Schwefel- und/oder Stickstoffatome enthalten, ausgewählte Gruppe stehen,
3) die Gruppen X gleich oder verschieden sind und für eine lineare oder verzweigte C₁- bis C₃₀-Alkylendiylgruppe, die in ihrer Kette ein oder mehrere Sauerstoff- und/oder Stickstoffatome enthalten kann, stehen,
4) Y für eine gesättigte oder ungesättigte, lineare oder verzweigte zweiwertige C₁- bis C₅₀-Alkylen-, -Arylen-, -Cycloalkylen-, -Alkylarylen- oder -Arylalkylengruppe, die ein oder mehrere Sauerstoff-, Schwefel- und/oder Stickstoffatome enthalten und/oder als Substituenten eines der folgenden Atome oder eine der folgenden Atomgruppen enthalten kann: Fluor, Hydroxy, C₃- bis C₈-Cycloalkyl, C₁- bis C₄₀-Alkyl, C₅- bis C₁₀-Aryl, Phenyl, das gegebenfalls durch 1 bis 3 C₁- bis C₃-Alkyl-, C₁- bis C₃-Hydroxyalkyl- und C₁- bis C₆-Aminoalkylgruppen substituiert ist, steht, oder
5) Y für eine Gruppe der Formel: 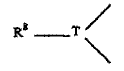 steht, in der
- T für eine lineare oder verzweigte, gesättigte oder ungesättigte drei- oder vierwertige C₃- bis C₂₄-Kohlenwasserstoffgruppe, die gegebenenfalls durch eine Polyorganosiloxankette substituiert ist und ein oder mehrere aus O, N und S ausgewählte Atome enthalten kann, steht oder T für ein aus N, P und Al ausgewähltes dreiwertiges Atom steht und
- R⁸ für eine lineare oder verzweigte C₁-C₅₀-Alkylgruppe oder eine Polyorganosiloxankette, die gegebenenfalls eine oder mehrere Ester-, Amid-, Urethan-, Thiocarbamat-, Harnstoff-, Thioharnstoff- und/oder Sulfonamidgruppen enthält und gegebenenfalls an eine andere Polymerkette gebunden sein kann, steht,
6) n für eine ganze Zahl im Bereich von 2 bis 500, vorzugsweise 2 bis 200 steht und m für eine ganze Zahl im Bereich von 50 bis 1000, vorzugsweise 50 bis 700 und noch besser 50 bis 200 steht, wobei "m" dem durchschnittlichen Polymerisationsgrad des Silikonteils des Silikonpolyamids entspricht.

5. Zusammensetzung nach dem vorhergehenden Anspruch, wobei X und/oder Y für eine Alkylengruppe, die in ihrem Alkylenteil mindestens eines der folgenden Elemente enthält:
1) 1 bis 5 Amid-, Harnstoff-, Urethan- oder Carbamatgruppen,
2) eine C₅- oder C₆-Cycloalkylgruppe und
3) eine Phenylengruppe, die gegebenenfalls durch 1 bis 3 gleiche oder verschiedene C₁- bis C₃-Alkylgruppen substituiert ist,
und gegebenenfalls durch mindestens ein Element aus der Gruppe bestehend aus:
- einer Hydroxygruppe,
- einer C₃- bis C₈-Cycloalkylgruppe,
- einer bis drei C₁- bis C₄₀-Alkylgruppen,
- einer Phenylgruppe, die gegebenenfalls durch eine bis drei C₁- bis C₃-Alkylgruppen substituiert ist,
- einer C₁- bis C₃-Hydroxyalkylgruppe und
- einer C₁- bis C₆-Aminoalkylgruppe substituiert ist, steht.

6. Zusammensetzung nach einem der Ansprüche 4 und 5, wobei R⁴, R⁵, R⁶ und R⁷ unabhängig für eine lineare oder verzweigte C₁- bis C₄₀-Alkylgruppe, vorzugsweise eine CH₃-, C₂H₅-, n-C₃H₇- oder Isopropylgruppe, eine Polyorganosiloxankette oder eine Phenylgruppe, die gegebenenfalls durch eine bis drei Methyl- oder Ethylgruppen substituiert ist, stehen.

7. Zusammensetzung nach einem der vorhergehenden Ansprüche, umfassend:
- mindestens ein erstes Silikonpolyamid mit mindestens einer Einheit der Formel (I) gemäß Anspruch 5, wobei m für das erste Polymer im Bereich von 50 bis 600, insbesondere 60 bis 400 und speziell 75 bis 200 liegt und noch spezieller etwa 120 beträgt, und
- mindestens ein zweites Silikonpolyamid mit mindestens einer zweiten Einheit der Formel (I) gemäß Anspruch 5, wobei G_{'}, G, R⁴, R⁵, R⁶, R⁷, X, Y und n die in Anspruch 5 angegebene Bedeutung besitzen und m für das zweite Polymer im Bereich von 5 bis 100 und insbesondere 10 bis 75 liegt und noch spezieller etwa 15 beträgt.

8. Zusammensetzung nach dem vorhergehenden Anspruch, **dadurch gekennzeichnet, dass** das Inden-Kohlenwasserstoffharz hydriert ist.

9. Zusammensetzung nach einem der Ansprüche 1 bis 8, **dadurch gekennzeichnet, dass** es sich bei dem Harz um ein aus hydrierten Inden/Methylstyrol/Styrol-Copolymeren ausgewähltes Indenharz handelt.

10. Zusammensetzung nach einem der vorhergehenden Ansprüche, **dadurch gekennzeichnet, dass** das Harz in einem Gehalt im Bereich von 0,1 bis 30 Gew.-%, bezogen auf das Gesamtgewicht der Zusammensetzung, vorzugsweise im Bereich von 0,3 bis 20 Gew.-% und weiter bevorzugt im Bereich von 0,5 bis 15 Gew.-% liegt.

11. Zusammensetzung nach einem der vorhergehenden Ansprüche, **dadurch gekennzeichnet, dass** das Silikonpolyamid/Harz-Massenverhältnis im Bereich von 2 bis 40 liegt.

12. Zusammensetzung nach einem der vorhergehenden Ansprüche, **dadurch gekennzeichnet, dass** sie mindestens ein Silikonöl, ein Esteröl oder ein Nichtsilikonöl umfasst.

13. Zusammensetzung nach einem der vorhergehenden Ansprüche, **dadurch gekennzeichnet, dass** sie eine Elastizität von mehr als 80% aufweist.

14. Verfahren zum Schminken der Haut und/oder der Lippen und/oder der Epithelanhanggebilde, bei dem man eine Zusammensetzung gemäß einem der vorhergehenden Ansprüche aufbringt.
